# EUROPEAN PATENT APPLICATION

(11) **EP 3 184 623 A1**
(43) Date of publication of application: **28.06.2017**
(21) Application number: 15834337.6
(22) Date of filing: 18.08.2015
(51) Int. Cl.: C12M 3/06, C12M 1/00, C12M 1/34, C12M 3/00

(54) **CELL CULTURE BAG, CELL CULTURE DEVICE, AND CELL CULTURE CONTAINER**

(30) Priority: 22.08.2014 JP 2014169026; 04.09.2014 JP 2014179721
(71) Applicant: OLYMPUS CORPORATION, Hachioji-shi Tokyo 192-8507 (JP)
(72) Inventor: KIMURA, Hiroyuki, Tokyo 151-0072 (JP); MINAMI, Tatsuya, Tokyo 151-0072 (JP); MAKARA, Yasunori, Tokyo 151-0072 (JP)
(74) Representative: Schicker, Silvia
(86) International application number: PCT/JP2015/073111
(87) International publication number: WO 2016/027800

(57) **Abstract**

A cell culture apparatus (100) according to the present invention includes a cell culture bag (1) and a culture tank (2). The cell culture bag (1) is composed of a bag-shaped film material capable of accommodating cells (A) and a culture medium (B) therein. At least a part of the bag-shaped film material is formed of a porous membrane that selectively allows a substance contained in the culture medium (B) to pass through but does not allow the cells (A) to pass through. The culture tank (2) retains the cell culture bag (1) in an immersed state in a solution (B).

## Description

### {Technical Field}

The present invention relates to cell culture bags, cell culture apparatuses, and cell culture containers.

### {Background Art}

With recent developments in stem-cell research and regenerative medicine, there are demands for preparing a large number of cells for clinical use. When culturing a large number of cells, culture bags composed of gas permeable materials or culture vats, such as bioreactors, are often used instead of using flasks or petri dishes for the culturing process (for example, see Patent Literature 1 and Patent Literature 2).

### {Citation List}

### {Patent Literature}

{PTL 1} Japanese Unexamined Patent Application, Publication No. 2000-125848
{PTL 2} Japanese Unexamined Patent Application, Publication No. 2011-188777

### {Summary of Invention}

### {Technical Problem}

During the culturing process, the cells take in components necessary for the growth, such as oxygen and nutrients, and release carbon dioxide and waste products. Therefore, when the cells are cultured over a long period of time, the culture medium needs to be replaced regularly due to degradation of the culture medium. The culture-medium replacing process is troublesome and costly to the operator and also involves the risk of the cell culture system being contaminated with, for example, bacteria. In order to prepare cells for clinical use, the culturing process has to comply with strict standards. Thus, it is effective to perform the culturing process in a closed system to reduce the risk of contamination and so on. However, it is extremely difficult to replace the culture medium in a closed system during the culturing process.

The present invention has been made in view of the circumstances described above, and an object thereof is to provide a cell culture bag, a cell culturing apparatus, and a cell culture container that can easily prevent degradation of a culture medium even in a culturing process in a closed system.

### {Solution to Problem}

In order to achieve the aforementioned object, the present invention provides the following solutions.

A first aspect of the present invention provides a cell culture apparatus including a cell culture bag and a culture tank that retains the cell culture bag in an immersed state in a solution. The cell culture bag is composed of a bag-shaped film material capable of accommodating cells and a culture medium therein. At least a part of the bag-shaped film material is formed of a porous membrane that selectively allows a substance contained in the culture medium to pass through but does not allow the cells to pass through.

According to the first aspect of the present invention, when culturing the cells by using the cell culture bag, the rate of degradation of the culture medium can be reduced even without the operator replacing the culture medium during the culturing period. Specifically, the cell culture apparatus according to this aspect can release, for example, waste products produced from the cells during the culturing process from the cell culture bag and supply substances necessary for the growth of the cells to the cell culture bag. Accordingly, degradation of the culture medium can be suppressed.

In the first aspect, the cell culture apparatus may further include a solution supplying means that supplies the solution to the culture tank and a solution discharging means that discharges the solution from the culture tank.

Accordingly, the solution within the culture tank can be kept in a fresh state, and the substance exchange efficiency between the culture medium within the cell culture bag and the solution within the culture tank can be enhanced.

In the first aspect, the cell culture apparatus may further include a controller that remotely controls the solution supplying means and the solution discharging means.

Accordingly, the solution within the culture tank can be remotely replaced at a freely-chosen timing, and degradation of the culture medium within the cell culture bag can be slowed down.

In the first aspect, the cell culture apparatus may further include a monitoring means that remotely monitors a state of the cells within the cell culture bag.

Accordingly, by remotely monitoring the condition of the cells, the solution within the culture tank can be replaced at a freely-chosen timing in accordance with the state of the cells, and degradation of the culture medium within the cell culture bag can be slowed down.

A second aspect of the present invention provides a cell culture bag composed of a bag-shaped film material capable of accommodating cells and a culture medium therein. At least a part of the bag-shaped film material is formed of a porous membrane that selectively allows a substance contained in the culture medium to pass through but does not allow the cells to pass through.

A third aspect of the present invention provides a cell culture container including the aforementioned cell culture bag and an accommodation bag that accommodates the cell culture bag therein and that is composed of a material having gas permeability.

A fourth aspect of the present invention provides a cell culture apparatus including a culture vat that retains cells and a culture medium therein and that is used for culturing the cells, and also including a dialysis bag composed of a bag-shaped film material capable of accommodating a dialysate solution therein. At least a part of the bag-shaped film material is formed of a porous membrane that selectively allows a substance contained in the culture medium to pass through but does not allow the cells to pass through. The dialysis bag is disposed inside the culture vat such that the dialysis bag is immersed in the culture medium.

According to the fourth aspect of the present invention, when culturing the cells in a closed space, the rate of degradation of the culture medium can be reduced even without the operator replacing the culture medium during the culturing period. Specifically, the cell culture apparatus according to this aspect can collect, for example, waste products produced from the cells during the culturing process into the dialysate solution within the dialysis bag, so as to suppress degradation of the culture medium.

In the fourth aspect, the culture vat may be a culture tank or a cell culture bag.

In the fourth aspect, the cell culture apparatus may further include a dialysate-solution supplying means that supplies the dialysate solution to the dialysis bag and a dialysate-solution discharging means that discharges the dialysate solution from the dialysis bag.

Accordingly, the solution within the dialysis bag can be kept in a fresh state, and the substance exchange efficiency between the culture medium within the culture vat and the solution within the dialysis bag can be enhanced.

In the fourth aspect, the cell culture apparatus may further include a plurality of the dialysis bags, and a plurality of the dialysate-solution supplying means and a plurality of the dialysate-solution discharging means that are provided respectively for the plurality of the dialysis bags. At least one of the plurality of the dialysis bags accommodates a dialysate solution having a different composition from a remaining one or more of the dialysis bags.

In the fourth aspect, the cell culture apparatus may further include a controller that remotely controls the dialysate-solution supplying means and the dialysate-solution discharging means.

Accordingly, the solution within the dialysis bag can be remotely replaced at a freely-chosen timing.

In the fourth aspect, the cell culture apparatus may further include a monitoring means that remotely monitors the state of the cells or the culture medium within the culture vat.

Accordingly, by remotely monitoring the condition of the cells or the culture medium, the solution within the dialysis bag can be replaced at a freely-chosen timing in accordance with the state of the cells or the culture medium, and degradation of the culture medium within the culture vat can be slowed down.

A fifth aspect of the present invention provides a cell culture container that retains a bag therein. The bag is composed of a bag-shaped film material that retains a dialysate solution therein. At least a part of the bag-shaped film material is formed of a porous membrane having the property that the porous membrane selectively allows a substance contained in a culture medium to pass through but does not allow cells to pass through.

### {Advantageous Effects of Invention}

According to the present invention, degradation of a culture medium can easily be prevented when culturing cells in a closed system by using a culture vat or a cell culture bag. Since this eliminates the need to replace the culture medium, the risk of the cell culture system being contaminated with, for example, bacteria can be reduced.

### {Brief Description of Drawings}

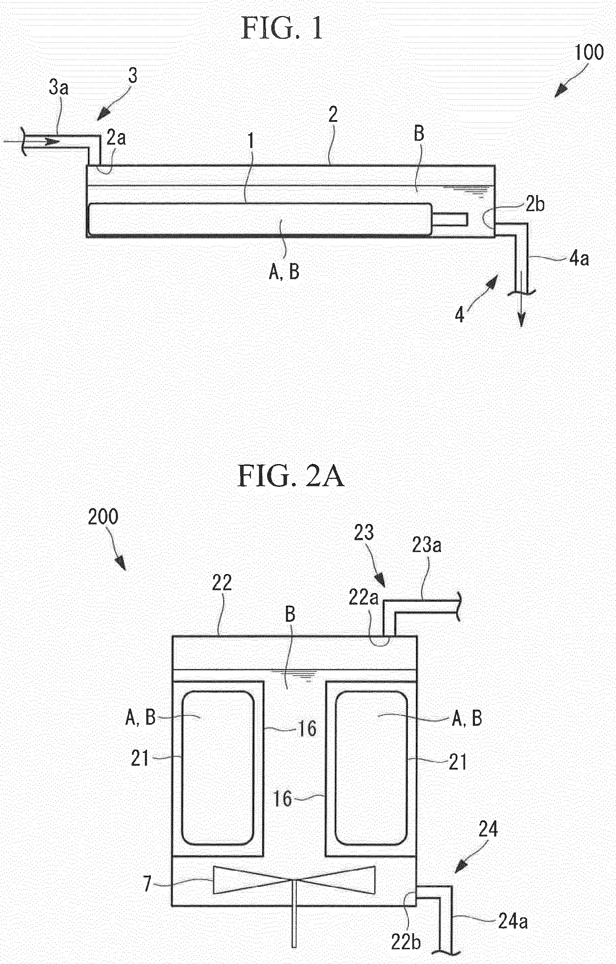

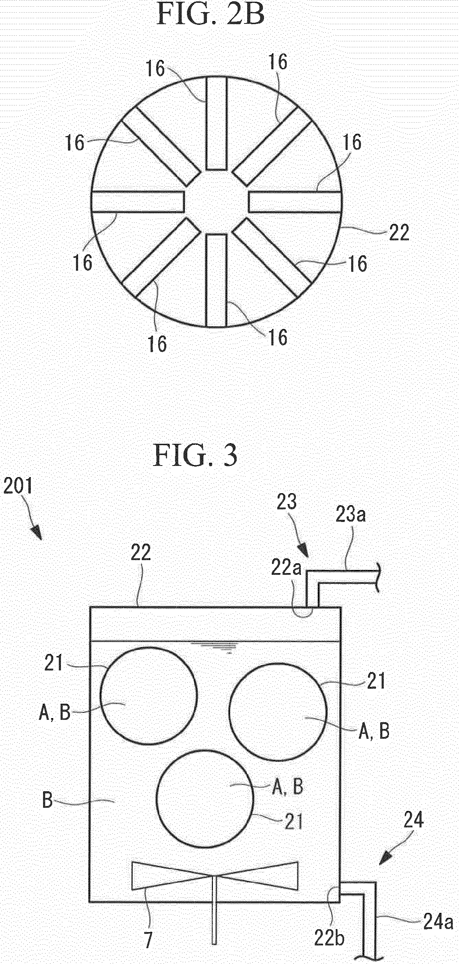

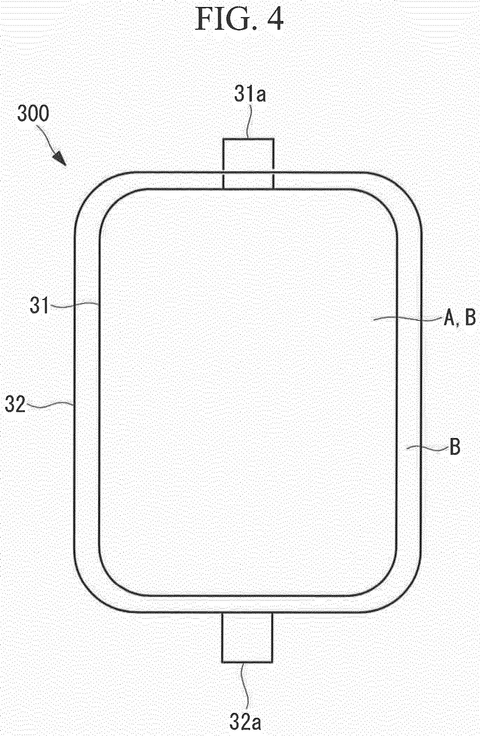

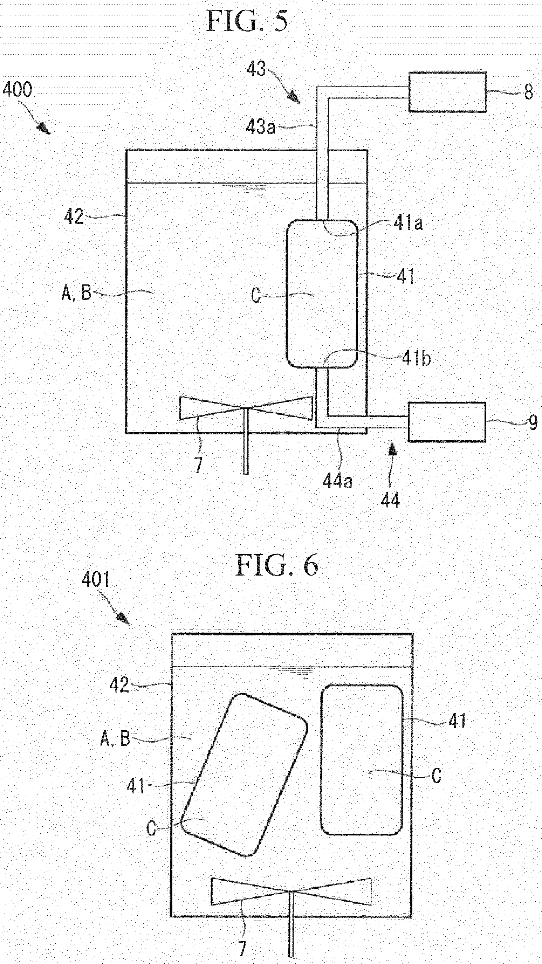

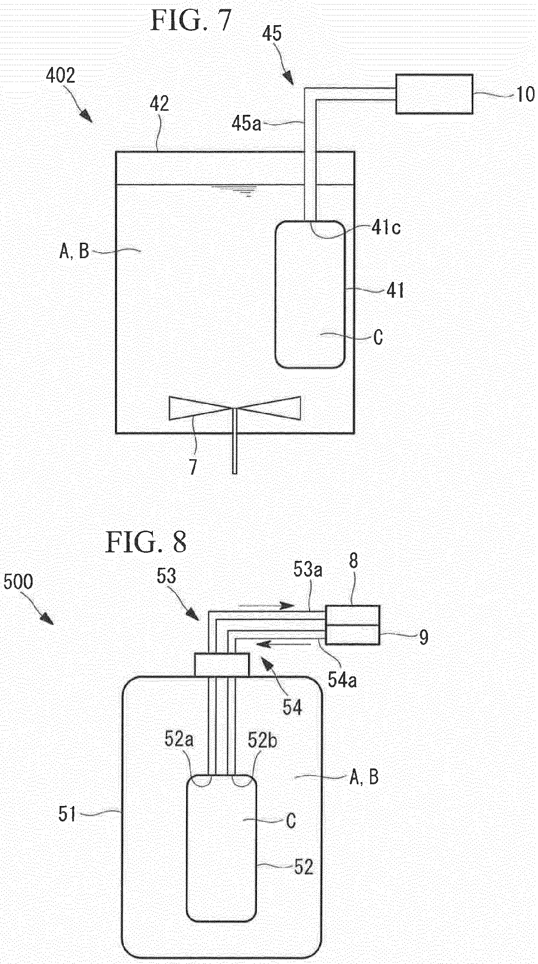

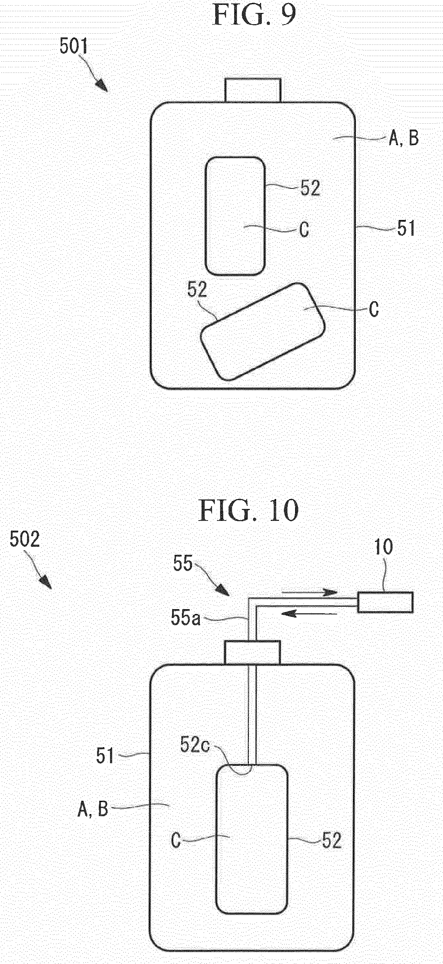

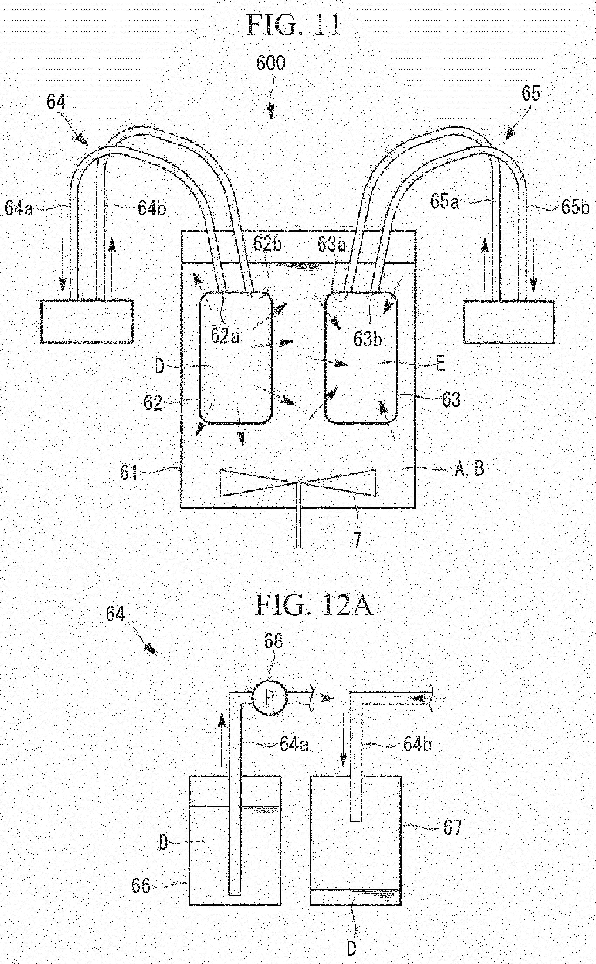

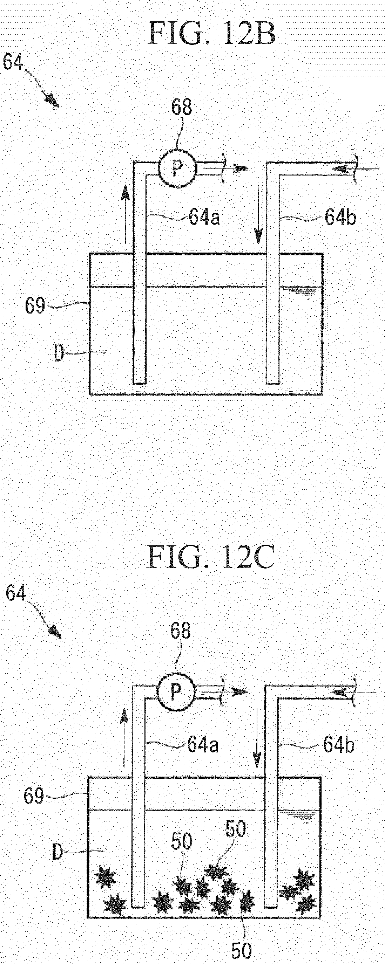

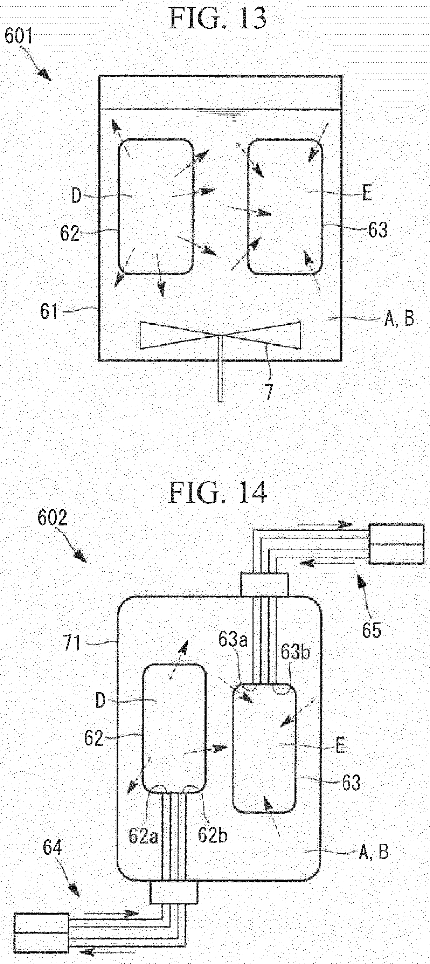

{Fig. 1} Fig. 1 schematically illustrates the configuration of a cell culture apparatus according to a first embodiment of the present invention.
{Fig. 2A} Fig. 2A schematically illustrates the configuration of a cell culture apparatus according to a second embodiment of the present invention and is a side view of the cell culture apparatus.
{Fig. 2B} Fig. 2B is a plan view of a culture tank of the cell culture apparatus in Fig. 2A, as viewed from above.
{Fig. 3} Fig. 3 schematically illustrates the configuration of a modification of the cell culture apparatus according to the second embodiment of the present invention.
{Fig. 4} Fig. 4 schematically illustrates the configuration of a cell culture container according to a third embodiment of the present invention.
{Fig. 5} Fig. 5 schematically illustrates the configuration of a cell culture apparatus according to a fourth embodiment of the present invention.
{Fig. 6} Fig. 6 schematically illustrates the configuration of a modification of the cell culture apparatus according to the fourth embodiment of the present invention.
{Fig. 7} Fig. 7 schematically illustrates the configuration of another modification of the cell culture apparatus according to the fourth embodiment of the present invention.
{Fig. 8} Fig. 8 schematically illustrates the configuration of a cell culture apparatus according to a fifth embodiment of the present invention.
{Fig. 9} Fig. 9 schematically illustrates the configuration of a modification of the cell culture apparatus according to the fifth embodiment of the present invention.
{Fig. 10} Fig. 10 schematically illustrates the configuration of another modification of the cell culture apparatus according to the fifth embodiment of the present invention.
{Fig. 11} Fig. 11 schematically illustrates the configuration of a cell culture apparatus according to a sixth embodiment of the present invention.
{Fig. 12A} Fig. 12A schematically illustrates the configuration of a first-solution replacing means of the cell culture apparatus in Fig. 11.
{Fig. 12B} Fig. 12B schematically illustrates the configuration of a modification of the first-solution replacing means in Fig. 12A.
{Fig. 12C} Fig. 12C schematically illustrates the configuration of another modification of the first-solution replacing means in Fig. 12A.
{Fig. 13} Fig. 13 schematically illustrates the configuration of a modification of the cell culture apparatus according to the sixth embodiment of the present invention.
{Fig. 14} Fig. 14 schematically illustrates the configuration of another modification of the cell culture apparatus according to the sixth embodiment of the present invention.

### {Description of Embodiments}

A cell culture bag, a cell culture apparatus, and a cell culture container according to embodiments of the present invention will be described below with reference to the drawings.

### {First Embodiment}

A cell culture apparatus 100 according to a first embodiment of the present invention will be described with reference to Fig. 1.

The cell culture apparatus 100 according to this embodiment is used when culturing cells by using a cell culture bag and has the configuration shown in Fig. 1.

The cell culture apparatus 100 includes a cell culture bag 1 in which cells A are cultured and a culture tank 2 that accommodates the cell culture bag 1 therein, and is set within an incubator (not shown) that maintains an environment suitable for culturing the cells A.

The cell culture bag 1 used in this apparatus 100 is a bag at least a part of which is formed of a porous membrane. The porous membrane is composed of a film material having pores that do not allow cells to pass through but allow components necessary for the growth of the cells A, such as oxygen and nutritive substances, as well as carbon dioxide and waste products released from the cells A, to freely pass through. Specifically, the cell culture bag 1 is composed of a bag-shaped film material that can accommodate the cells A and a culture medium B therein, and at least a part of the bag-shaped film material is formed of a porous membrane having the property that it selectively allows the substances contained in the culture medium B to pass through but does not allow the cells A to pass through (in other words, the porous membrane has a pore diameter smaller than the diameter of the cells A and larger than the diameters of predetermined substances contained in the culture medium B).

The culture tank 2 has a structure that can accommodate the cell culture bag 1 therein and is supplied with the culture medium (cell culture solution) B such that the cell culture bag 1 is immersed therein, whereby the culture tank 2 can culture the cells within the cell culture bag 1 in a closed fashion.

Furthermore, the cell culture apparatus 100 includes a solution supplying means 3 and a solution discharging means 4 that are connected to the culture tank 2. The solution supplying means 3 can supply the culture medium B to the culture tank 2 in a closed fashion, and the solution discharging means 4 can discharge the culture medium B from the culture tank 2 in a closed fashion.

The culture tank 2 has a supply port 2a and a discharge port 2b. The solution supplying means 3 includes, for example, a tubular member 3a, such as a tube, one end of which is connected to the supply port 2a, and the solution discharging means 4 includes, for example, a tubular member 4a, such as a tube, one end of which is connected to the discharge port 2b. The other end of the tubular member 3a is connected to a culture-medium container (not shown) that retains a fresh culture medium therein, and the culture medium B is supplied from the culture-medium container to the culture tank 2 via the tubular member 3a by means of a liquid feed pump (not shown) disposed at an intermediate position in the tubular member 3a. The other end of the tubular member 4a is connected to a waste container (not shown), and the culture medium B is discharged from the culture tank 2 to the waste container via the tubular member 4a by means of a liquid feed pump (not shown) disposed at an intermediate position in the tubular member 4a. A negative-pressure applying means (not shown) may apply negative pressure to the discharge port 2b so that the culture medium B is discharged from the culture tank 2 to the waste container via the tubular member 4a.

Next, an example of a procedure for replacing the culture medium B by using the cell culture apparatus 100 according to this embodiment will be described.

A user of this apparatus 100 first prepares the cell culture bag 1 filled with the cells A to be cultured and the culture medium B and accommodates the cell culture bag 1 inside the culture tank 2. Then, the culture medium B is supplied into the culture tank 2 such that the cell culture bag 1 is immersed therein, and the cell culture apparatus 100 is set within the incubator. Accordingly, carbon dioxide and waste products released from the cells A within the cell culture bag 1 pass through the film material constituting the cell culture bag 1 so as to be released to the culture medium B within the culture tank 2, and oxygen and nutritive substances consumed by the cells A within the cell culture bag 1 pass through the film material constituting the cell culture bag 1 from the culture medium B within the culture tank 2 so as to be resupplied into the cell culture bag 1.

The solution supplying means 3 appropriately supplies fresh culture medium B into the culture tank 2, and the solution discharging means 4 appropriately discharges the culture medium B from the culture tank 2, so that the culture medium B within the culture tank 2 is replaceable. This allows the culture medium B within the culture tank 2 to be maintained in a fresh state and facilitates the discharging of carbon dioxide and waste products from the cell culture bag 1 and the supplying of oxygen and nutritive substances to the cell culture bag 1.

It is preferable that the supply port 2a via which the culture medium B is supplied to the culture tank 2 by means of the solution supplying means 3 be set above the liquid level of the culture medium B within the culture tank 2 so that the culture medium B is supplied in a dripping manner. Accordingly, the culture medium B can be prevented from flowing backward to the solution supplying means 3, thereby preventing the culture medium B to be supplied from the solution supplying means 3 from being contaminated.

### {Second Embodiment}

Next, a cell culture apparatus 200 according to a second embodiment of the present invention will be described with reference to Figs. 2A to 3.

The cell culture apparatus 200 according to this embodiment is used when culturing cells by using a cell culture bag and has the configuration shown in Figs. 2A and 2B.

The cell culture apparatus 200 includes cell culture bags 21 in which cells A are cultured and a culture tank 22 that accommodates the plurality of cell culture bags 21 therein.

Each of the cell culture bags 21 used in this apparatus 200 is similar to the cell culture bag 1 according to the first embodiment in that at least a part of the bag is formed of a porous membrane. The porous membrane is composed of a film material having pores that do not allow the cells A to pass through but allow components necessary for the growth of the cells A, such as oxygen and nutritive substances, as well as carbon dioxide and waste products released from the cells A, to freely pass through. Specifically, each cell culture bag 1 is composed of a bag-shaped film material that can accommodate the cells A and a culture medium B therein, and at least a part of the bag-shaped film material is formed of a porous membrane having the property that it selectively allows the substances contained in the culture medium B to pass through but does not allow the cells A to pass through.

The culture tank 22 has a structure that can accommodate the plurality of cell culture bags 21 therein and is supplied with the culture medium (cell culture solution) B such that the cell culture bags 21 are immersed therein. The culture tank 22 can maintain the internal environment in a state suitable for culturing cells and can culture the cells A within the cell culture bag 1 in a closed fashion. Furthermore, the culture tank 22 includes a solution supplying means 23 and a solution discharging means 24. For example, the solution supplying means 23 and the solution discharging means 24 are similar to the solution supplying means 3 and the solution discharging means 4 according to the first embodiment in that they are respectively provided with tubular members 23a and 24a, such as tubes, one ends of which are connected to a supply port 22a and a discharge port 22b, respectively, of the culture tank 22. The solution supplying means 23 can supply the culture medium B to the culture tank 22 in a closed fashion, and the solution discharging means 24 can discharge the culture medium B from the culture tank 22 in a closed fashion.

Next, an example of a procedure for replacing the culture medium B by using the cell culture apparatus 200 according to this embodiment will be described.

A user of this apparatus 200 first prepares the cell culture bags 21 filled with the cells A to be cultured and the culture medium B and sets the cell culture bags 21 in respective bag holders 16. The bag holders 16 can be secured at predetermined positions inside the culture tank 22 and secure the cell culture bags 21 set in the bag holders 16 within the culture tank 22. Then, the culture medium B is supplied into the culture tank 22 such that the cell culture bags 21 are immersed therein, and the internal environment is set in a closed system by, for example, covering the culture tank 22 with a lid.

Accordingly, for example, carbon dioxide and waste products released from the cells A within the cell culture bags 21 pass through the film material constituting each cell culture bag 21 so as to be released to the culture medium B within the culture tank 22, and oxygen and nutritive substances consumed by the cells A within each cell culture bag 21 pass through the film material constituting the cell culture bag 21 from the culture medium B within the culture tank 22 so as to be resupplied into the cell culture bag 21.

The solution supplying means 23 appropriately supplies fresh culture medium B into the culture tank 22, and the solution discharging means 24 appropriately discharges the culture medium B from the culture tank 22, so that the culture medium B within the culture tank 22 is replaceable. This allows the culture medium B within the culture tank 22 to be appropriately maintained in a fresh state and facilitates the discharging of carbon dioxide and waste products from the cell culture bags 21 and the supplying of oxygen and nutritive substances to the cell culture bags 21.

It is preferable that the supply port 22a via which the culture medium B is supplied to the culture tank 22 by means of the solution supplying means 23 be set above the liquid level of the culture medium B within the culture tank 22 so that the culture medium B is supplied in a dripping manner. Accordingly, the culture medium B can be prevented from flowing backward to the solution supplying means 23, thereby preventing the culture medium B to be supplied from the solution supplying means 23 from being contaminated.

Although the cell culture bags 21 are secured inside the culture tank 22 in this embodiment, an example in which the cell culture bags 21 are not secured inside the culture tank 22, as in a cell culture apparatus 201 shown in Fig. 3, is also possible.

### {Third Embodiment}

Next, a cell culture container 300 according to a third embodiment of the present invention will be described with reference to Fig. 4.

The cell culture container 300 according to this embodiment is used when culturing cells A by using a cell culture bag and has the configuration shown in Fig. 4.

The cell culture container 300 includes a cell culture bag 31 in which the cells A are cultured and an accommodation bag 32 that accommodates the cell culture bag 31 therein.

The cell culture bag 31 used in this container 300 is similar to the cell culture bag 1 according to the first embodiment in that at least a part of the bag is formed of a porous membrane. The porous membrane is composed of a film material having pores that do not allow the cells A to pass through but allow components necessary for the growth of the cells A, such as oxygen and nutritive substances, as well as carbon dioxide and waste products released from the cells A, to freely pass through. Specifically, the cell culture bag 31 is composed of a bag-shaped film material that can accommodate the cells A and a culture medium B therein, and at least a part of the bag-shaped film material is formed of a porous membrane having the property that it selectively allows the substances contained in the culture medium B to pass through but does not allow the cells A to pass through.

The cell culture bag 31 has an opening 31a, and the culture medium B and the cells A can be taken in and out through the opening 31a. When the culture medium B and the cells A are not to be taken in and out, the opening 31a can be closed with, for example, a lid.

The accommodation bag 32 has the shape of a bag that can accommodate the cell culture bag 31 therein and can retain the culture medium B such that the cell culture bag 31 is immersed therein.

The accommodation bag 32 has an opening 32a, and the culture medium B can be taken in and out through the opening 32a. When the culture medium B is not to be taken in and out, the opening 32a can be closed with, for example, a lid.

The opening 31a of the cell culture bag 31 extends through the accommodation bag 32 without impairing the sealability of the accommodation bag 32.

It is preferable that the accommodation bag 32 be composed of a material with high gas permeability. Examples of the material of the accommodation bag 32 include LLDPE (linear low-density polyethylene), LDPE (low-density polyethylene), EVA (ethylene-vinyl acetate copolymer resin), ionomer resin, and polyethylene-based resin.

Next, an example of a procedure for replacing the culture medium B by using the cell culture container 300 according to this embodiment will be described.

A user of this container 300 supplies the cells A to be cultured and the culture medium B into the cell culture bag 31 through the opening 31a and also supplies the culture medium B into the accommodation bag 32 through the opening 32a. In this state, the container 300 is set within an incubator that maintains an environment suitable for culturing the cells A. Accordingly, carbon dioxide and waste products released from the cells A within the cell culture bag 31 pass through the film material constituting the cell culture bag 31 so as to be released to the culture medium B within the accommodation bag 32, and oxygen and nutritive substances consumed by the cells A within the cell culture bag 31 pass through the film material constituting the cell culture bag 31 from the culture medium B within the accommodation bag 32 so as to be resupplied into the cell culture bag 31.

This allows the culture medium B within the accommodation bag 32 to be maintained in a fresh state by being appropriately replaced via the opening 32a, and facilitates the discharging of carbon dioxide and waste products from the cell culture bag 31 and the supplying of oxygen and nutritive substances to the cell culture bag 31.

Although the culture tank 2 or 22 or the accommodation bag 32 is filled with the culture medium B in each of the first to third embodiments described above, the solution that fills the culture tank 2 or 22 or the accommodation bag 32 is not limited to the culture medium B and may be any kind of solution, such as a dialysate solution, which can perform an exchange, with the culture medium B within the cell culture bag 1, 21, or 31, between oxygen and carbon dioxide and between waste products and substances necessary for the growth of cells and does not have an adverse effect on the growth of the cells A. One example that can be used is a physiological saline solution that contains oxygen and nutritive substances to be supplied to the culture medium B within the cell culture bag 1, 21, or 31 and the osmotic pressure of which has been adjusted to allow for high substance exchange efficiency. Another example that can be used is a solution that does not contain nutritive substances and the osmotic pressure of which has been adjusted to collect waste products. Another example that can be used is a solution with a composition in which an expensive substance has been removed from the culture-medium component. This is advantageous in terms of costs since the expensive substance contained in the culture medium B would not be consumed.

In each of the first and second embodiments described above, a stirring means 7 that stirs the culture medium B may be provided within the culture tank 2 or 22, as shown in Figs. 2A and 3. This allows for enhanced substance exchange efficiency with respect to the culture medium B inside and outside the cell culture bag 1 or 21.

In each of the first and second embodiments described above, an oxygen supplying means (not shown) for supplying oxygen to a solution, such as the culture medium B, to be supplied into the culture tank 2 or 22 by means of the solution supplying means 3 or 23 may further be provided. The oxygen supplying means may be set within the culture tank 2 or 22. An example of the oxygen supplying means includes a means for feeding oxygen or oxygen-containing gas to a solution, such as the culture medium B, by bubbling.

In each of the first to third embodiments described above, a monitoring means (not shown) that can remotely monitor the state of the solution within the culture tank 2 or 22 or the accommodation bag 32 or the state of the cells A within the cell culture bag 1, 21, or 31 may be provided.

Furthermore, a controller (not shown) that remotely controls the solution supplying means 3 or 23 and the solution discharging means 4 or 24 may be provided. Based on the state of the solution or the cells A monitored by the monitoring means, the controller may control the solution supplying means 3 or 23 and the solution discharging means 4 or 24. Accordingly, the culture medium B within the culture tank 2 or 22 is replaced while monitoring the state of the cells A cultured in a closed system, thereby preventing degradation of the culture medium B within the cell culture bag 1, 21, or 31. The remote control in this case may be performed in a wireless or wired manner.

The porous membrane used in each of the first to third embodiments described above may be appropriately selected in accordance with the sizes (molecular weights) of substances that are allowed to pass through the porous membrane. Examples of the porous membrane include a dialysis membrane and a semipermeable membrane.

### {Fourth Embodiment}

Next, a cell culture apparatus 400 according to a fourth embodiment of the present invention will be described with reference to Figs. 5 to 7.

The cell culture apparatus 400 according to this embodiment is used when culturing cells A in a culture vat, such as a bioreactor, and has the configuration shown in Fig. 5.

The cell culture apparatus 400 includes a culture tank 42 that retains the cells A and the culture medium B therein and that can culture the cells A by maintaining the internal environment in a state suitable for culturing cells, and also includes a dialysis bag 41 that is set inside the culture tank 42 and that retains a dialysate solution C therein.

The dialysis bag 41 used in this apparatus 400 is a bag at least a part of which is formed of a porous membrane. The porous membrane is composed of a film material having pores that do not allow the cells A to pass through but allow carbon dioxide and waste products released from the cells A to freely pass through. Specifically, the dialysis bag 41 is composed of a bag-shaped film material that can retain the dialysate solution C therein, and at least a part of the bag-shaped film material is formed of a porous membrane having the property that it selectively allows the substances contained in the culture medium B to pass through but does not allow the cells A to pass through.

The dialysis bag 41 has a supply port 41a for supplying the dialysate solution C into the bag and a discharge port 41b for discharging the dialysate solution C from the bag. The supply port 41a is connected to a dialysate-solution supplying means 43 so that the dialysate solution C can be supplied into the dialysis bag 41 from the outside via the supply port 41a. The discharge port 41b is connected to a dialysate-solution discharging means 44 so that the dialysate solution C in the dialysis bag 41 can be discharged outside via the discharge port 41b.

The dialysate-solution supplying means 43 includes, for example, a tubular member 43a, such as a tube, one end of which is connected to the supply port 41a, and the dialysate-solution discharging means 44 includes, for example, a tubular member 44a, such as a tube, one end of which is connected to the discharge port 41b. The other end of the tubular member 43a is connected to a culture medium container 8 that retains a fresh culture medium B therein, and the culture medium B is supplied from the culture medium container 8 to the dialysis bag 41 via the tubular member 43a by means of a liquid feed pump (not shown) disposed at an intermediate position in the tubular member 43a. The other end of the tubular member 44a is connected to a waste container 9, and the culture medium B is discharged from the dialysis bag 41 to the waste container 9 via the tubular member 44a by means of a liquid feed pump (not shown) disposed at an intermediate position in the tubular member 44a. A negative-pressure applying means (not shown) may apply negative pressure to the discharge port 41b so that the culture medium B is discharged from the dialysis bag 41 to the waste container 9 via the tubular member 44a.

In order to enhance the substance exchange efficiency between the culture medium B within the culture tank 42 and the dialysate solution C within the dialysis bag 41, it is more advantageous that the contact area between the culture medium B within the culture tank 42 and the porous membrane of the dialysis bag 41 be larger. Therefore, it is preferable that the entire dialysis bag 41 be formed of a porous membrane. Furthermore, since it is more advantageous that the dialysis bag 41 have a larger surface area, it is preferable that the dialysis bag 41 have an irregular shape, such as protrusions. Alternatively, the dialysis bag 41 may have a long and narrow shape (like a tube) and be disposed in the form of a flow channel within the culture tank 42 (e.g., the inner wall thereof).

The dialysate solution C within the dialysis bag 41 has a composition in which the osmotic pressure has been adjusted so that carbon dioxide and waste products released from the cells A in the culture medium B within the culture tank 42 move toward the dialysate solution C within the dialysis bag 41 via the porous membrane.

The culture tank 42 retains the cells A and the culture medium (cell culture solution) B therein and can culture the cells A in a closed fashion. The dialysis bag 41 is disposed within the culture tank 42 such that the dialysis bag 41 is immersed in the culture medium B, and the dialysate-solution supplying means 43 and the dialysate-solution discharging means 44 connected to the dialysis bag 41 are configured not to impair the closed state within the culture tank 42 during the culturing process. Therefore, during the culturing process, the dialysate solution C can be supplied into the dialysis bag 41 in a closed fashion, and the dialysate solution C can be discharged from the dialysis bag 41 in a closed fashion.

Next, an example of a procedure for culturing the cells A by using the cell culture apparatus 400 according to this embodiment will be described.

A user of this apparatus 400 first sets the culture medium B and the cells A to be cultured inside the culture tank 42 and disposes the dialysis bag 41 at a position where it is immersed in the culture medium B within the culture tank 42. Then, the dialysate solution C is supplied to the dialysis bag 41 by means of the dialysate-solution supplying means 43 so as to fill the dialysis bag 41 with the dialysate solution C. Subsequently, the culturing process commences under conditions suitable for culturing the cells A. Accordingly, carbon dioxide and waste products released from the cells A within the culture tank 42 pass through the porous membrane of the dialysis bag 41 so as to move to the dialysate solution C within the dialysis bag 41.

After discharging the dialysate solution C from the dialysis bag 41 at an appropriate timing by means of the dialysate-solution discharging means 44, a fresh dialysate solution C is supplied into the dialysis bag 41 by means of the dialysate-solution supplying means 43, thereby replacing the dialysate solution C within the dialysis bag 41.

The dialysate solution C within the dialysis bag 41 may be replaced at a timing desired by the user. Alternatively, the dialysate solution C may be replaced regularly at certain intervals, or the supplying and discharging of the dialysate solution C may be performed continuously at a desired flow rate.

The dialysate solution C within the dialysis bag 41 may alternatively be a solution that contains oxygen and substances necessary for the growth of the cells A and that has a composition in which the osmotic pressure has been adjusted so that carbon dioxide and waste products released from the cells A in the culture medium B within the culture tank 42 move toward the dialysate solution C within the dialysis bag 41 via the porous membrane while the oxygen and the substances simultaneously move toward the culture medium B within the culture tank 42 via the porous membrane. In this case, the porous membrane of the dialysis bag 41 has pores that allow the oxygen and the substances necessary for the growth of the cells A to pass through.

In this embodiment, the dialysis bag 41 may be configured to be not connected to a supplying means or a discharging means, as in a cell culture apparatus 401 shown in Fig. 6, such that the dialysate solution C is not replaced during the culturing period. In that case, the dialysis bag 41 may be set in a floating state within the culture medium B without being secured in the culture tank 42.

Although the supply port 41a and the discharge port 41b are provided separately in this embodiment, a supply/discharge port 41c serving as a combination of the supply port 41a and the discharge port 41b may be provided, as in a cell culture apparatus 402 shown in Fig. 7, such that the dialysate solution C is supplied and discharged via the supply/discharge port 41c. In this case, a supplying/discharging means 45 connected to the supply/discharge port 41c and serving as a combination of the dialysate-solution supplying means and the dialysate-solution discharging means may be employed. Specifically, the supplying/discharging means 45 includes a single tubular member 45a, such as a tube, one end of which is connected to the supply/discharge port 41c. The other end of the tubular member 45a is connected to a single container 10. Alternatively, the dialysate-solution supplying means and the dialysate-solution discharging means may be connected to the same supply/discharge port 41c via a tubular member, such as a tube, branching off from an intermediate position.

In this embodiment, a plurality of the dialysis bags 41 may be provided within the culture tank 42. This allows for enhanced substance exchange efficiency between the culture medium B within the culture tank 42 and the dialysate solution C within each dialysis bag 41.

In this embodiment, a stirring means 7 that stirs the culture medium B may be provided within the culture tank 42, as shown in Figs. 5 to 7. This allows for enhanced substance exchange efficiency between the culture medium B within the culture tank 42 and the dialysate solution C within the dialysis bag 41.

In this embodiment, the culture tank 42 may be provided with a culture-medium supplying means (not shown) that can supply the culture medium B to the culture tank 42 in a closed fashion.

Moreover, an oxygen supplying means (not shown) for supplying oxygen to the culture medium B to be supplied to the culture tank 42 by means of the culture-medium supplying means may be further provided. The oxygen supplying means may be set within the culture tank 42. An example of the oxygen supplying means includes a means for feeding oxygen or oxygen-containing gas to a culture solution by, for example, bubbling.

1 {Fifth Embodiment}

Next, a cell culture apparatus 500 according to a fifth embodiment of the present invention will be described with reference to Figs. 8 to 10.

The cell culture apparatus 500 according to this embodiment is used when culturing cells A by using a cell culture bag and has the configuration shown in Fig. 8.

The cell culture apparatus 500 includes a cell culture bag 51 in which the cells A are cultured and a dialysis bag 52 that is set inside the cell culture bag 51 and that retains the dialysate solution C therein, and is set within an incubator (not shown) that maintains an environment suitable for culturing the cells A.

The dialysis bag 52 used in this apparatus 500 is similar to the dialysis bag 41 according to the fourth embodiment in that at least a part of the bag is formed of a porous membrane. The porous membrane is composed of a film material having pores that do not allow the cells A to pass through but allow, for example, carbon dioxide and waste products released from the cells A to freely pass through. Specifically, the dialysis bag 52 is composed of a bag-shaped film material that can retain the dialysate solution C therein, and at least a part of the bag-shaped film material is formed of a porous membrane having the property that it selectively allows the substances contained in the culture medium B to pass through but does not allow the cells A to pass through.

The dialysis bag 52 has a supply port 52a for supplying the dialysate solution C into the bag and a discharge port 52b for discharging the dialysate solution C from the bag. The supply port 52a is connected to a dialysate-solution supplying means 53 so that the dialysate solution C can be supplied into the dialysis bag 52 from the outside via the supply port 52a. The discharge port 52b is connected to a dialysate-solution discharging means 54 so that the dialysate solution C in the dialysis bag 52 can be discharged outside via the discharge port 52b. Similar to the dialysate-solution supplying means 43 according to the fourth embodiment, the dialysate-solution supplying means 53 includes a tubular member 53a, such as a tube, for connecting the supply port 52a and the culture medium container 8. Similar to the dialysate-solution discharging means 44 according to the fourth embodiment, the dialysate-solution discharging means 54 includes a tubular member 54a, such as a tube, for connecting the discharge port 52b and the waste container 9.

In order to enhance the substance exchange efficiency between the culture medium B within the cell culture bag 51 and the dialysate solution C within the dialysis bag 52, it is more advantageous that the contact area between the culture medium B within the cell culture bag 51 and the porous membrane of the dialysis bag 52 be larger. Therefore, it is preferable that the entire dialysis bag 52 be formed of a porous membrane. Furthermore, since it is more advantageous that the dialysis bag 52 have a larger surface area, it is preferable that the dialysis bag 52 have an irregular shape, such as protrusions. Alternatively, the dialysis bag 52 may have a long and narrow shape (like a tube) and be disposed in the form of a flow channel within the cell culture bag 51.

The dialysate solution C within the dialysis bag 52 has a composition in which the osmotic pressure has been adjusted so that carbon dioxide and waste products released from the cells A in the culture medium B within the cell culture bag 51 move toward the dialysate solution C within the dialysis bag 52 via the porous membrane.

The cell culture bag 51 retains the cells A and the culture medium (cell culture solution) B therein and can culture the cells A in a closed fashion. The dialysis bag 52 is disposed within the cell culture bag 51 such that the dialysis bag 52 is immersed in the culture medium B. The dialysate-solution supplying means 53 and the dialysate-solution discharging means 54 connected to the dialysis bag 52 are configured not to impair the closed state within the cell culture bag 51 during the culturing process. Therefore, during the culturing process, the dialysate solution C can be supplied into the dialysis bag 52 in a closed fashion, and the dialysate solution C can be discharged from the dialysis bag 52 in a closed fashion.

Next, an example of a procedure for culturing the cells A by using the cell culture apparatus 500 according to this embodiment will be described.

A user of this apparatus 500 first sets the culture medium B and the cells A to be cultured inside the cell culture bag 51 and disposes the dialysis bag 52 at a position where it is immersed in the culture medium B within the cell culture bag 51. Then, the dialysate solution C is supplied to the dialysis bag 52 by means of the dialysate-solution supplying means 53 so as to fill the dialysis bag 52 with the dialysate solution C. In order to culture the cells A in conditions suitable for culturing the cells A, the cell culture apparatus 500 is set within an incubator (not shown). Accordingly, carbon dioxide and waste products released from the cells A within the cell culture bag 51 pass through the porous membrane of the dialysis bag 52 so as to move to the dialysate solution C within the dialysis bag 52.

After discharging the dialysate solution C from the dialysis bag 52 at an appropriate timing by means of the dialysate-solution discharging means 54, the dialysate solution C is supplied into the dialysis bag 52 by means of the dialysate-solution supplying means 53, thereby replacing the dialysate solution C within the dialysis bag 52.

The dialysate solution C within the dialysis bag 52 may be replaced at a timing desired by the user. Alternatively, the dialysate solution C may be replaced regularly at certain intervals, or the supplying and discharging of the dialysate solution C may be performed continuously at a desired flow rate.

The dialysate solution C within the dialysis bag 52 may alternatively be a solution that contains oxygen and substances necessary for the growth of the cells A and that has a composition in which the osmotic pressure has been adjusted so that carbon dioxide and waste products released from the cells A in the culture medium B within the cell culture bag 51 move toward the dialysate solution C within the dialysis bag 52 via the porous membrane while the oxygen and the substances simultaneously move toward the culture medium B within the cell culture bag 51 via the porous membrane. In this case, the porous membrane of the dialysis bag 52 has pores that allow the oxygen and the substances necessary for the growth of the cells A to pass through.

In this embodiment, the dialysis bag 52 may be configured to be not connected to a dialysate-solution supplying means or a dialysate-solution discharging means, as in a cell culture apparatus 501 shown in Fig. 9, such that the dialysate solution C is not replaced during the culturing period. In that case, the dialysis bag 52 may be set in a floating state within the culture medium B without being secured in the cell culture bag 51.

As an alternative to the separately-provided supply port 52a and discharge port 52b in this embodiment, a supply/discharge port 52c serving as a combination of a supply port and a discharge port may be provided, as in a cell culture apparatus 502 shown in Fig. 10, and the dialysate solution C may be supplied and discharged via the supply/discharge port 52c.

In this case, a supplying/discharging means 55 connected to the supply/discharge port 52c and serving as a combination of a supplying means and a discharging means may be employed. Specifically, the supplying/discharging means 55 includes a single tubular member 55a, such as a tube, one end of which is connected to the supply/discharge port 52c. The other end of the tubular member 55a is connected to a single container 10. Alternatively, the dialysate-solution supplying means and the dialysate-solution discharging means may be connected to the same supply/discharge port 52c via a tubular member, such as a tube, branching off from an intermediate position.

In this embodiment, a plurality of the dialysis bags 52 may be provided within the cell culture bag 51. This allows for enhanced substance exchange efficiency between the culture medium B within the cell culture bag 51 and the dialysate solution C within each dialysis bag 52.

In this embodiment, a culture-medium supplying means (not shown) that can supply the culture medium B to the cell culture bag 51 in a closed fashion may be provided.

Moreover, an oxygen supplying means (not shown) for supplying oxygen to the culture medium B to be supplied to the cell culture bag 51 by means of the culture-medium supplying means may be further provided. An example of the oxygen supplying means includes a means for feeding oxygen or oxygen-containing gas to a culture solution by, for example, bubbling.

Although the cell culture bag 51 is employed in this embodiment, a different cell culture container, such as a flask or a petri dish, may be used in place of the cell culture bag 51.

### {Sixth Embodiment}

Next, a cell culture apparatus 600 according to a sixth embodiment of the present invention will be described with reference to Figs. 11 to 14.

The cell culture apparatus 600 according to this embodiment is used when culturing cells A in a culture vat, such as a bioreactor, and has the configuration shown in Fig. 11.

The cell culture apparatus 600 includes a culture tank 61 that retains the cells A and the culture medium B therein and that can culture the cells A by maintaining the internal environment in a state suitable for culturing cells, a first dialysis bag 62 that is set inside the culture tank 61 and that retains a first solution D therein, and a second dialysis bag 63 that is set inside the culture tank 61 and that retains a second solution E therein.

The first dialysis bag 62 used in this apparatus 600 is a bag at least a part of which is formed of a porous membrane. The porous membrane is composed of a film material having pores that do not allow the cells A to pass through but at least allow carbon dioxide and waste products released from the cells A to freely pass through. Specifically, the first dialysis bag 62 is composed of a bag-shaped film material that can retain the solution D therein, and at least a part of the bag-shaped film material is formed of a porous membrane having the property that it selectively allows the substances contained in the culture medium B to pass through but does not allow the cells A to pass through.

The first dialysis bag 62 has a supply port 62a for supplying the first solution D into the bag and a discharge port 62b for discharging the first solution D from the bag. The supply port 62a and the discharge port 62b are connected to a first-solution replacing means 64, and the first solution D can be supplied into the first dialysis bag 62 and be discharged therefrom.

The first solution D within the first dialysis bag 62 has a composition in which the osmotic pressure has been adjusted so that carbon dioxide produced in the culture medium B in the culture tank 61 and waste products released from the cells A move toward the first solution D within the first dialysis bag 62 via the porous membrane.

In order to enhance the substance exchange efficiency between the culture medium B and the first solution D, it is more advantageous that the contact area between the culture medium B within the culture tank 61 and the porous membrane of the first dialysis bag 62 be larger. Therefore, it is preferable that the entire first dialysis bag 62 be formed of a porous membrane. Furthermore, since it is more advantageous that the first dialysis bag 62 have a larger surface area, it is preferable that the first dialysis bag 62 have an irregular shape, such as protrusions. Alternatively, the first dialysis bag 62 may have a long and narrow shape (like a tube) and be disposed in the form of a flow channel within the culture tank 61 (e.g., the inner wall thereof).

The first-solution replacing means 64 will be described with reference to Figs. 12A to 12C.

The first-solution replacing means 64 functions as a dialysate-solution supplying means for supplying the first solution D and a dialysate-solution discharging means for discharging the first solution D.

Fig. 12A illustrates an example of the first-solution replacing means 64 including two tubular members 64a and 64b, such as tubes, respectively connected to the supply port 62a and the discharge port 62b, a first-solution container 66 that retains the first solution D to be supplied to the culture tank 61, and a waste container 67 that retains the first solution D discharged from the culture tank 61. The first-solution container 66 is coupled to the supply port 62a of the first dialysis bag 62 via the tubular member 64a, and the waste container 67 is coupled to the discharge port 62b of the first dialysis bag 62 via the tubular member 64b. For example, a liquid feed pump 68 is set in the tubular member 64a so that the first solution D can be sequentially fed from the first-solution container 46 to the first dialysis bag 62 and then to the waste container 67.

Fig. 12B illustrates an example provided with a container 69 obtained by combining the first-solution container 66 and the waste container 67 in Fig. 12A into a single unit. The container 69 is coupled to the supply port 62a and the discharge port 62b of the first dialysis bag 62 via the tubular members 64a and 64b, such as tubes. For example, the liquid feed pump 68 is set in the tubular member 64a so that the first solution D can circulate between the container 69 and the first dialysis bag 62.

As shown in Fig. 12C, an absorptive material 50, such as a porous material, which absorbs waste products, may be set within the container 69.

The position where the liquid feed pump 68 is set may be appropriately determined in accordance with the culture system.

The second dialysis bag 63 used in this apparatus 600 is a bag at least a part of which is formed of a porous membrane. The porous membrane is formed of a film material having pores that do not allow the cells A to pass through but at least allow, for example, oxygen and nutritive substances necessary for the growth of the cells A to freely pass through. Specifically, the second dialysis bag 63 is composed of a bag-shaped film material that can retain the solution E therein, and at least a part of the bag-shaped film material is formed of a porous membrane having the property that it selectively allows the substances contained in the culture medium B to pass through but does not allow the cells A to pass through.

The second dialysis bag 63 has a supply port 63a for supplying the second solution E into the bag and a discharge port 63b for discharging the second solution E from the bag. The supply port 63a and the discharge port 63b are connected to a second-solution replacing means 65, and the second solution E can be supplied into the second dialysis bag 63 and discharged therefrom via tubular members 65a and 65b, such as tubes.

The second solution E within the second dialysis bag 63 contains oxygen and nutritive substances necessary for the cells A and has a composition in which the osmotic pressure has been adjusted so that the oxygen and the nutritive substances move toward the culture medium B in the culture tank 61 via the porous membrane.

In order to enhance the substance exchange efficiency between the culture medium B and the second solution E, it is more advantageous that the contact area between the culture medium B within the culture tank 61 and the porous membrane of the second dialysis bag 63 be larger. Therefore, it is preferable that the entire second dialysis bag 63 be formed of a porous membrane. Furthermore, since it is more advantageous that the second dialysis bag 63 have a larger surface area, it is preferable that the second dialysis bag 63 have an irregular shape, such as protrusions. Alternatively, the second dialysis bag 63 may have a long and narrow shape (like a tube) and be disposed in the form of a flow channel within the culture tank 61 (e.g., the inner wall thereof).

It is preferable that the culture tank 61 retain the cells A and the culture medium (cell culture solution) B therein and culture the cells A in a closed fashion. The first dialysis bag 62 is disposed within the culture tank 61 such that the first dialysis bag 62 is immersed in the culture medium B. The first-solution replacing means 64 connected to the first dialysis bag 62 is configured not to impair the closed state within the culture tank 61 during the culturing process. Furthermore, the second dialysis bag 63 is disposed within the culture tank 61 such that the second dialysis bag 63 is immersed in the culture medium B. The second-solution replacing means 65 connected to the second dialysis bag 63 is configured not to impair the closed state within the culture tank 61 during the culturing process.

Therefore, during the culturing process, the first solution D can be supplied into the first dialysis bag 62 in a closed fashion, and the first solution D can be discharged from the first dialysis bag 62 in a closed fashion. Likewise, during the culturing process, the second solution E can be supplied into the second dialysis bag 63 in a closed fashion, and the second solution E can be discharged from the second dialysis bag 63 in a closed fashion.

The second-solution replacing means 65 may be similar to the first-solution replacing means 64 and preferably has the configuration shown in Fig. 12A.

Next, an example of a procedure for culturing the cells A by using the cell culture apparatus 600 according to this embodiment will be described.

A user of this apparatus 600 first sets the culture medium B and the cells A to be cultured inside the culture tank 61 and disposes the first dialysis bag 62 and the second dialysis bag 63 at positions where they are immersed in the culture medium B within the culture tank 61. Then, the first dialysis bag 62 and the second dialysis bag 63 are respectively connected to the first-solution replacing means 64 and the second-solution replacing means 65. Subsequently, the first dialysis bag 62 is filled with the first solution D by means of the first-solution replacing means 64, and the second dialysis bag 63 is filled with the second solution E by means of the second-solution replacing means 65. In this state, the culturing process commences under conditions suitable for culturing the cells A within the culture tank 61.

Accordingly, carbon dioxide and waste products released from the cells A within the culture tank 61 pass through the porous membrane of the first dialysis bag 62 so as to move to the first solution D within the first dialysis bag 62.

By supplying and discharging the first solution D within the first dialysis bag 62 at an appropriate timing by means of the first-solution replacing means 64, the first solution D within the first dialysis bag 62 is replaced, so that the carbon dioxide and the waste products released from the cells A can be discharged from the culture tank 61.

The oxygen and the nutritive substances necessary for the cells A in the second solution E pass through the porous membrane of the second dialysis bag 63 so as to move into the culture tank 61.

By supplying and discharging the second solution E within the second dialysis bag 63 at an appropriate timing by means of the second-solution replacing means 65, the second solution E within the second dialysis bag 63 is replaced, so that the oxygen and the nutritive substances necessary for the cells A can be supplied into the culture tank 61.

The solutions D and E within the respective dialysis bags 62 and 63 may be replaced at timings desired by the user. Alternatively, the solutions D and E may be replaced regularly at certain intervals, or the supplying and discharging of the solutions D and E may be performed continuously at a desired flow rate.

In this embodiment, the first dialysis bag 62 and the second dialysis bag 63 may be configured to be not connected to the first-solution replacing means 64 and the second-solution replacing means 65, as in a cell culture apparatus 601 shown in Fig. 13, such that the solutions D and E are not replaced during the culturing period. In that case, the first dialysis bag 62 and the second dialysis bag 63 may be set in a floating state within the culture medium B without being secured in the culture tank 61.

In this embodiment, a plurality of the first dialysis bags 62 and a plurality of the second dialysis bags 63 may be provided within the culture tank 61. This allows for enhanced substance exchange efficiency between the culture medium B within the culture tank 61 and the solutions D and E within the first dialysis bags 62 and the second dialysis bags 63.

In this case, an absorptive material, such as a porous material, which absorbs waste products, may be set within each first dialysis bag 62.

In this embodiment, a cell culture bag 71 may be employed in place of the culture tank 61, as in a cell culture apparatus 602 shown in Fig. 14. In this case, the cell culture bag 71 may be set within an incubator so as to maintain a state suitable for culturing cells.

This embodiment can provide a cell culture apparatus including a culture vat (culture tank) for retaining cells and a culture medium therein and for culturing the cells and a plurality of dialysis bags each of which is composed of a bag-shaped film material capable of accommodating a dialysate solution (solution) therein. At least a part of the bag-shaped film material is formed of a porous membrane that selectively allows substances contained in the culture medium to pass through but does not allow the cells to pass through. The plurality of dialysis bags are disposed within the culture vat (culture tank) such that the dialysis bags are immersed in the culture medium. At least one of the plurality of dialysis bags accommodates a dialysate solution (solution) having a different composition from that in the other dialysis bag.

It is preferable that the plurality of dialysis bags accommodate a dialysate solution (solution) having a composition in which the osmotic pressure has been adjusted so that carbon dioxide and waste products released from the cells move from the culture medium in the culture vat to the corresponding dialysis bag and also accommodate a dialysate solution (solution) having a composition in which the osmotic pressure has been adjusted so that oxygen and nutritive substances necessary for the cells move from the corresponding dialysis bag to the culture medium in the culture vat.

Specifically, the plurality of dialysis bags preferably include a dialysis bag for collecting the carbon dioxide and the waste products released from the cells from the culture medium in the culture vat (culture tank) and a dialysis bag for releasing the oxygen and the nutritive substances necessary for the cells to the culture medium in the culture vat (culture tank).

Furthermore, the plurality of dialysis bags may each be connected to a solution replacing means via a tubular member, such as a tube, so that the dialysate solution (solution) can be supplied from and discharged to the outside by means of the solution replacing means.

A modification of this embodiment is a cell culture apparatus not equipped with the first dialysis bag that retains the first solution.

Specifically, the cell culture apparatus includes a culture vat (culture tank) for retaining cells and a culture medium therein and for culturing the cells and a dialysis bag formed of a porous membrane composed of a bag-shaped film material capable of accommodating a dialysate solution (solution) therein. At least a part of the bag-shaped film material is formed of a porous membrane that selectively allows substances contained in the culture medium to pass through but does not allow the cells to pass through. The dialysis bag is disposed within the culture vat (culture tank) such that the dialysis bag is immersed in the culture medium. Moreover, the dialysis bag accommodates a dialysate solution (solution) having a composition in which the osmotic pressure has been adjusted so that oxygen and nutritive substances necessary for the cells move from the dialysis bag to the culture medium in the culture vat (culture tank).

The dialysis bag may be connected to a solution replacing means (i.e., a supplying means and a discharging means) via a tubular member, such as a tube, so that the dialysate solution (solution) can be supplied from and discharged to the outside by means of the solution replacing means. Alternatively, the dialysis bag need not be connected to a solution replacing means, such that the dialysate solution (solution) is not replaced during the culturing period. A plurality of the dialysis bags may be provided.

The culture tank may alternatively be a cell culture bag. In this case, the cell culture bag may be set within an incubator so as to maintain a state suitable for culturing cells.

With the cell culture apparatus according to this modification, the oxygen and nutritive substances consumed by the cells during the culturing process can be continuously supplied to the culture system.

In the fourth to sixth embodiments described above, a culture medium identical to the culture medium B within the culture tank 42 or 61 or the cell culture bag 51 may be used as the dialysate solution C or the first or second solution D or E that fills the dialysis bag 41, 52, 62, or 63.

In the fourth to sixth embodiments described above, a monitoring means that can remotely monitor the state of the cells A or the culture medium B within the culture tank 42 or 61 or the cell culture bag 51 may be provided.

Furthermore, a controller that remotely controls the dialysate-solution supplying means and the dialysate-solution discharging means may be provided. Based on the state of the cells A or the culture medium B monitored by the monitoring means, the controller may control the dialysate-solution supplying means and the dialysate-solution discharging means. Accordingly, the dialysate solution C, D, or E within the corresponding dialysis bag 41, 52, 62, or 63 is replaced while monitoring the state of the cells A cultured in a closed system or the culture medium B, thereby preventing degradation of the culture medium B within the culture tank 42 or 61 or the cell culture bag 51. The remote control in this case may be performed in a wireless or wired manner.

In the fourth to sixth embodiments described above, a material that absorbs waste products may be set within the dialysis bag 41, 52, 62, or 63. An example of the material that absorbs waste products includes a porous material having micro-pores, such as activated carbon.

The porous membrane used in each of the first to sixth embodiments described above may be appropriately selected in accordance with the sizes (molecular weights) of substances that are allowed to pass through the porous membrane. Examples of the porous membrane that can be used include a dialysis membrane and a semipermeable membrane. The porous membrane may be, for example, a cellulose-based membrane (such as a recycled cellulose membrane, a modified-surface recycled cellulose membrane, or a cellulose acetate membrane) and a synthetic-polymer-based membrane (such as polyacrylonitrile, polymethyl methacrylate, an ethylene-vinylalcohol copolymer, polysulfone, polyamide, or a polyester-based polymer alloy).

An example of the controller in the present invention includes a computer having a CPU and a memory. The computer may be a general-purpose computer, such as a personal computer (PC), or may be a dedicated computer. The memory is, for example, a computer-readable nonvolatile storage medium and stores a control program. The CPU executes the control program stored in the memory so as to realize the functions of the controller described above.

### {Reference Signs List}

100, 200, 201, 400, 401, 402, 500, 501, 502, 600, 601, 602 cell culture apparatus
300 cell culture container
1, 21, 31, 51, 71 cell culture bag
2, 22, 42, 61 culture tank
3, 23 solution supplying means
4, 24 solution discharging means
43, 53, 64 dialysate-solution supplying means
44, 54, 64 dialysate-solution discharging means
2a, 22a, 41a, 52a, 62a, 63a supply port
2b, 22b, 41b, 52b, 62b, 63b discharge port
16 bag holder
7 stirring means
32 accommodation bag
41, 52, 62, 63 dialysis bag
45, 55 supplying/discharging means
41c, 52c supply/discharge port

## Claims

1. A cell culture bag composed of a bag-shaped film material capable of accommodating cells and a culture medium therein,
wherein at least a part of the bag-shaped film material is formed of a porous membrane that selectively allows a substance contained in the culture medium to pass through but does not allow the cells to pass through.

2. A cell culture apparatus comprising:
the cell culture bag according to Claim 1; and
a culture tank that retains the cell culture bag in an immersed state in a solution.

3. The cell culture apparatus according to Claim 2, further comprising:
a solution supplying means that supplies the solution to the culture tank; and
a solution discharging means that discharges the solution from the culture tank.

4. The cell culture apparatus according to Claim 3, further comprising:
a controller that remotely controls the solution supplying means and the solution discharging means.

5. The cell culture apparatus according to any one of Claims 2 to 4, further comprising:
a monitoring means that remotely monitors a state of the cells within the cell culture bag.

6. A cell culture container comprising:
the cell culture bag according to Claim 1; and
an accommodation bag that accommodates the cell culture bag therein and that is composed of a material having gas permeability.

7. A cell culture apparatus comprising:
a culture vat that retains cells and a culture medium therein and that is used for culturing the cells; and
a dialysis bag composed of a bag-shaped film material capable of accommodating a dialysate solution therein, wherein at least a part of the bag-shaped film material is formed of a porous membrane that selectively allows a substance contained in the culture medium to pass through but does not allow the cells to pass through,
wherein the dialysis bag is disposed inside the culture vat such that the dialysis bag is immersed in the culture medium.

8. The cell culture apparatus according to Claim 7,
wherein the culture vat is a culture tank or a cell culture bag.

9. The cell culture apparatus according to Claim 7 or 8, further comprising:
a dialysate-solution supplying means that supplies the dialysate solution to the dialysis bag; and
a dialysate-solution discharging means that discharges the dialysate solution from the dialysis bag.

10. The cell culture apparatus according to Claim 9, further comprising:
a plurality of the dialysis bags; and
a plurality of the dialysate-solution supplying means and a plurality of the dialysate-solution discharging means that are provided respectively for the plurality of the dialysis bags,
wherein at least one of the plurality of the dialysis bags accommodates a dialysate solution having a different composition from a remaining one or more of the dialysis bags.

11. The cell culture apparatus according to Claim 9 or 10, further comprising:
a controller that remotely controls the dialysate-solution supplying means and the dialysate-solution discharging means.

12. The cell culture apparatus according to any one of Claims 7 to 11, further comprising:
a monitoring means that remotely monitors a state of the cells within the culture vat.

13. The cell culture apparatus according to any one of Claims 7 to 12,
wherein the porous membrane is a dialysis membrane or a semipermeable membrane.

14. A cell culture container that retains a bag therein,
wherein the bag is composed of a bag-shaped film material that retains a dialysate solution therein, and wherein at least a part of the bag-shaped film material is formed of a porous membrane having the property that the porous membrane selectively allows a substance contained in a culture medium to pass through but does not allow cells to pass through.

15. The cell culture container according to Claim 14,
wherein the porous membrane is a dialysis membrane or a semipermeable membrane.
